# EUROPÄISCHE PATENTSCHRIFT

(11) **EP 1 613 364 B1**
(45) Veröffentlichungstag und Bekanntmachung des Hinweises auf die Patenterteilung: **16.01.2013**
(21) Anmeldenummer: 04713503.3
(22) Anmeldetag: 23.02.2004
(51) Int. Cl.: A61F 2/28, A61L 27/38, A61F 2/30, A61L 27/36, A61L 27/56, A61F 2/38

(54) **PRÄPARAT ZUR REPARATUR VON KNORPELGEWEBE, INSBESONDERE VON GELENKKNORPEL-DEFEKTEN**
PREPARATION FOR REPAIRING CARTILAGE TISSUE, ESPECIALLY ARTICULAR CARTILAGE DEFECTS
PREPARATION POUR REPARER UN TISSU CARTILAGINEUX, EN PARTICULIER DES DEFAUTS DE CARTILAGE ARTICULAIRE

(30) Priorität: 26.02.2003 CH 296032003
(43) Veröffentlichungstag der Anmeldung: 11.01.2006
(73) Patentinhaber: Zimmer Orthobiologics, Inc., Austin, Texas 78726-4050 (US)
(72) Erfinder: BERARDI VILEI, Simona, CH-6527 Lodrino (CH); BITTMANN, Peter, CH-8057 Zürich (CH); WAGNER, Philipp, CH-1196 Gland (CH); FRENZ, Martin, CH-3110 Muensingen (CH)
(74) Vertreter: Frei, Alexandra Sarah
(86) Internationale Anmeldenummer: PCT/CH2004/000093
(87) Internationale Veröffentlichungsnummer: WO 2004/075940

(56) Entgegenhaltungen:
- EP-A- 1 270 025
- WO-A-01/17463
- WO-A-02/070030
- US-A- 5 007 934
- US-A- 5 713 374
- US-A- 6 080 194
- US-B1- 6 454 811
- ZÜGER B J ET AL: "Laser solder welding of articular cartilage: tensile strength and chondrocyte viability." LASERS IN SURGERY AND MEDICINE. 2001, Bd. 28, Nr. 5, 2001, Seiten 427-434, XP002285204 ISSN: 0196-8092

## Beschreibung

Die Erfindung liegt auf dem Gebiete der Medizinaltechnik und betrifft ein Präparat nach dem Oberbegriff des ersten, unabhängigen Patentanspruchs. Das Präparat dient zur Reparatur von Knorpelgewebe, insbesondere zur Reparatur von Defekten in Gelenkknorpel.

Es ist bekannt, enchondrale Gelenkknorpeldefekte, das heisst, Gelenkknorpeldefekte, die nur den Knorpel, nicht aber das darunterliegende Knochengewebe betreffen, zu reparieren, indem Zellen mit einem chondrogenen Potential (Chondrozyten, Stammzellen etc.) in Suspension, in einem Gel oder in einer vorteilhafterweise resorbierbaren Matrix in die Defektstelle eingebracht werden. Um die in die Defektstelle eingebrachten Materialien unmittelbar nach der Operation und während einer Heilungsphase sicher darin zu halten, wird beispielsweise vorgeschlagen, ein Stück Knochenhaut über die Defektstelle zu nähen. Matrixmaterialien für die Reparatur von enchondralen Defekten sind beispielsweise beschrieben in den Publikationen US-6352558 und WO-99/19005 (Geistlich). Es handelt sich dabei um mehrschichtige Membrane auf Kollagenbasis, die auf die Grösse der zu reparierenden Defektstelle geschnitten und in die Defektstelle eingebracht werden. Diese Membrane weisen eine Matrixschicht auf, die insbesondere aus Kollagen II besteht und eine schwammartige Struktur hat, sich also für die Ansiedlung von Chondrozyten oder anderen Zellen mit einem chondrogenen Potential eignet. Ferner weisen die Membrane eine Barriereschicht auf, die bei der Implantation gegen die subchondrale Knochenplatte gerichtet wird. Diese Barriereschicht besteht insbesondere aus Kollagen I und III und ihre gegenüber der Matrixschicht bedeutend dichtere Struktur soll das Einwandern von Zellen aus dem Knochengewebe in die Reparaturstelle und damit auch deren Vaskularisierung verhindern. Um die genannte Membran in der Defektstelle festzuhalten, wird über diese, wie oben erwähnt, ein Stück Knochenhaut genäht. Es wird auch vorgeschlagen, die Membran beidseitig mit einer Barriereschicht zu versehen, wodurch sie auf der Aussenseite mechanische Eigenschaften erhält, die es erlauben, die Membran selbst im umliegenden Knorpelgewebe festzunähen.

Auch zur Reparatur von osteochondralen Gelenkknorpeldefekten, solchen Defekten also, die nicht nur das Knorpelgewebe sondern auch das unter dem Knorplegewebe liegende Knochengewebe betreffen, wird die Implantation von vorteilhafterweise resorbierbaren Präparaten vorgeschlagen. Derartige Präparate sind beispielsweise beschrieben in den Publikationen WO-93/15694 und US-5152791. Sie weisen einen Knorpelteil und einen Knochenteil auf, wobei die beiden Teile sich voneinander unterscheiden bezüglich Porosität und/oder bezüglich Material. Die Präparate werden üblicherweise in entsprechend vorbereiteten Öffnungen (Bohrungen) implantiert, wobei die Öffnung einen etwas kleineren Querschnitt aufweist als das Präparat. Der dadurch entstehende, sogenannte Pressfit im mechanisch festeren Knochengewebe und die grössere Tiefe der Verankerung im Vergleich zu in enchondralen Defekten positionierten Präparaten genügen üblicherweise, um das Implantat in der Defektstelle derart festzuhalten, dass es auch bei Belastung des Gelenkes an der Stelle verbleibt.

Aus der obigen Beschreibung geht hervor, dass es gemäss dem Stande der Technik mindestens für Implantate für enchondrale Gelenkknorpeldefekte zur Erreichung einer genügenden, primären Stabilität, die ein Verbleiben des Implantates in der Defektstelle nach der Implantation und während der Heilungsphase gewährleistet, entweder eine Entnahme von Knochenhaut braucht, also eine relativ aufwendige Operation oder dass die Matrix entsprechend verändert werden muss (äussere Barriereschicht), wobei diese Veränderung gegebenenfalls für andere Funktionen nicht vorteilhaft sein kann.

Ferner zeigt es sich, dass zusätzlich zum Problem der primären Stabilität der genannten Implantate auch das Problem der Integration oder Verwachsung des Implantates bzw, des an seiner Stelle während einer Heilungsphase entstehenden Reparaturgewebes im umliegenden Gewebe zu lösen ist. Es zeigt sich, dass die Integration im Knorpelgewebe und in der subchondralen Knochenplatte (bzw. in entsprechendem Reparaturgewebe) in vielen Fällen nicht befriedigend ausfällt.

Weitere Matrixmaterialien für die Reparatur von enchondralen und osseochondralen Defekten sind beispielsweise beschrieben in den Publikationen EP 2'70'025, welches eine Kompositenstruktur, mit einem porösen Keramikteil und einem offenporigen Polymerteil mit einer Zwischenstruktur offenbart; in US 6'454'811, welches Komposite offenbart für die Regenerierung von Gewebe insbesondere von Gewebe zwischen Knochen und Knorpel mit einem Knochenteil und einem Knochenteil; und in WO-01/17463, wo ein resorbierbares Implantatgerüst für die Regeneration eines Knorpels in Gelenken mit kräftigen Seitenteilen enthaltend Zellen offenbart wird.

Die Erfindung stellt sich nun die Aufgabe, ein Präparate zur Reparatur von Defektstellen in Knorpelgewebe, insbesondere zur Reparatur von Gelenkknorpel zu schaffen, welches Präparat gegenüber bekannten Präparaten, die denselben Zweck erfüllen, eine Verbesserung: bezüglich Integration und/oder bezüglich primärer Stabilität bringt, wobei beide Verbesserungen nicht mit einer Erhöhung des Operations-Aufwandes verbunden sein sollen. Die Erfindung bezieht sich auf die Reparatur von Knorpelgewebe, wobei das erfindungsgemässe Präparat nur einen Knorpelgewebe ersetzenden Teil (Knorpelteil) aufweist und zur Reparatur von enchondralen Defekten dient oder zusätzlich einen an den Knorpelteil anschliessenden, Knochengewebe ersetzenden Teil (Knochenteil) aufweist und zur Reparatur von osteochondralen Defekten dient.

Die gestellte Aufgabe wird gelöst durch das Präparat, wie es in den Patentansprüchen definiert ist.

Im Unterschied zu entsprechenden Präparaten gemäss dem Stande der Technik ist der Knorpelteil des erfindungsgemässen Präparates nicht homogen, sondern weist in einem peripheren Bereich (nach der Implantation an umliegendem Knorpelgewebe anliegender Bereich) und/oder in einem basalen Bereich (Bereich, der nach der Implantation an der subchondralen Knochenplatte anliegt oder bei einem Implantat für einen osteochondralen Defekt an den Knochenteil des Implantates angrenzt) Eigenschaften auf, die der Integration und/oder der primären Stabilität des Präparates und nicht der Verhinderung der Einwanderung von Zellen dienlich sind, während der übrige, zentrale Bereich keine solchen Eigenschaften aufweist sondern für eine optimale Regeneration von Knorpelgewebe ausgerüstet ist. Es zeigt sich, dass mit einem derartigen, bereichsweise für verschiedene Reparatur-Funktionen ausgerüsteten Knorpelteil Verbesserungen bezüglich Integration und/oder primärer Stabilität erreicht werden können, ohne dass dadurch die Bildung von funktionsfähigem Reparaturgewebe im zentralen Bereich beeinträchtigt wird.

Der Knorpelteil des erfindungsgemässen Präparates weist eine mindestens teilweise resorbierbare Matrix auf, die beispielsweise auf Kollagen basiert. In dieser Matrix werden vor der Implantation Zellen mit einem chondrogenen Potential angesiedelt und gegebenenfalls vor der Implantation in vitro vorkultiviert oder die Matrix wird ohne Zellen implantiert und wird dann von aus umliegendem Gewebe zuwandemden Reparaturzellen besiedelt. Die im peripheren und/oder basalen Bereich des Knorpelteils gegenüber dem zentralen Bereich des Knorpelteils verschiedenen Eigenschaften gehören zu mindestens einer der folgenden Gruppen: Eigenschaften der Matrixstruktur, Eigenschaften der Matrixzusammensetzung oder Eigenschaften der in der Matrix angesiedelten Zellen.

Die Erfindung basiert insbesondere auf dem Befund, dass es für eine gute Integration des Reparaturgewebes in umliegendem Gewebe (Knorpel und/oder Knochen) vorteilhaft ist, wenn im Integrationsbereich wie bei einer Wundheilung relativ wenig differenzierte Zellen (z.B. Stammzellen), die aber umso beweglicher und umso vermehrungsfähiger sind, zur Verfügung stehen, während es für eine möglichst rasche und erfolgreiche Regeneration von Knorpelgewebe im zentralen Bereich vorteilhafter ist, wenn möglichst gezielt differenzierte Zellen, also Chondrozyten oder bezüglich Differenzierung nicht weit von Chondrozyten entfernte Zellen zur Verfügung stehen, welche Zellen bekannterweise wenig beweglich sind und sich nur wenig vermehren können.

Eine Bildung von gutem Knorpel-Reparaturgewebe und gleichzeitig eine gute Integration dieses Knorpel-Reparaturgewebes im umliegenden Gewebe wird mit der Differenzierung von vor der Implantation in der Matrix angesiedelten Zellen oder von nach der Implantation in die Matrix eingewanderten Zellen im gewünschten Sinne erreicht, indem der basale und/oder periphere Bereich anders mit Faktoren ausgestattet als der zentrale Teil: Im zentralen Bereich sind sofort wirkende, eine Zelldifferenzierung zu Chondrozyten fördernde Faktoren enthalten, welche im peripheren und/oder basalen Bereich fehlen oder in einer verzögert wirkenden Form enthalten sind

Zusätzlich oder anstelle einer entsprechenden Auswahl von Zellen kann beispielsweise die folgende, weitere Massnahmen herangezogen werden:_Zur Erleichterung der Zellbeweglichkeit und Zellvermehrung wird die Porosität der Matrix im peripheren und/oder basalen Bereich höher gewühlt als im zentralen Bereich, wobei in diesem Falle gegebenenfalls zusätzliche Massnahmen bezüglich primärer Stabilität notwendig werden.

Der folgenden detaillierten Beschreibung der Erfindung dienen auch die folgenden Figuren. Diese zeigen:
- **Figur 1**: eine beispielhafte Ausführungsform des erfindungsgemässen Präparats zur Implantation in einer enchondralen Defektstelle in einem Gelenk und die entsprechend vorbereitete Defektstelle;
- **Figur 2**: ein mit einem erfindungsgemässen Präparat reparierter Meniskus;
- **Figuren 3 bis 5**: weitere, beispielhafte Ausführungsformen des erfindungsgemässen Präparates im Schnitt.

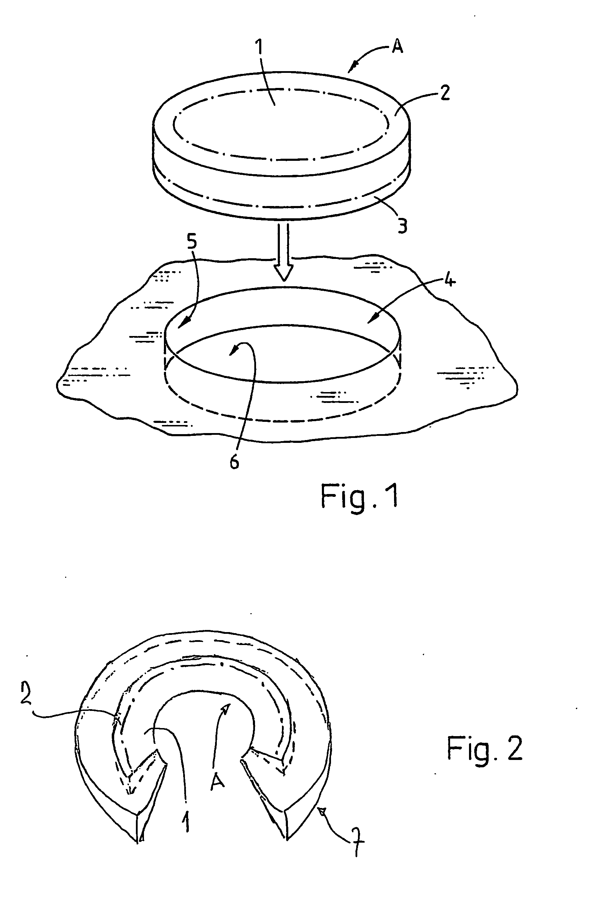

**Figur 1** zeigt eine beispielhafte Ausführungsform des erfindungsgemässen Präparates. Dieses dient zur Reparatur eines enchondralen Defektes in einem Gelenk und weist aus diesem Grunde nur einen Knorpelteil A auf. Das Präparat, bzw. der Knorpelteil des Präparates weist einen zentralen Bereich 1, einen peripheren Bereich 2 und einen basalen Bereich 3 auf. Das Präparat wird mit dem basalen Bereich 3 voran in die gegebenenfalls entsprechend vorbereitete Defektstelle 4 implantiert. Die Defektstelle reicht durch die Knorpelschicht 5 bis auf die subchondrale Knochenplatte 6. In implantiertem Zustand liegt der basale Bereich 3 des Präparates auf der nativen subchondralen Knochenplatte 6 auf.

Das Präparat hat eine Dicke, die an die Dicke der zu reparierenden Knorpelschicht 5 angepasst ist, ist also für die Reparatur von humanem Gelenkknorpel etwa 1 bis 3 mm dick. Die flächige Ausdehnung des Präparates ist an die Defektstelle anzupassen; das Präparat hat beispielsweise die Form einer Kreisscheibe mit einem Durchmesser von 3 bis 10 mm. Der periphere Bereich 2 und der basale Bereich 3 des Präparates umfassen je nach Ausführungsform einen grösseren oder einen kleineren Teil des gesamten Volumens des Präparates, in den meisten Fällen wird aber der zentrale Bereich 1 den grössten Teil des Volumens einnehmen.

Insbesondere im Falle von Präparaten für die Reparatur von enchondralen Defektstellen in Gelenken ist es vorteilhaft, sowohl den peripheren Bereich 2 als auch den basalen Bereich 3 anders als den zentralen Bereich 1 zu gestalten und/oder auszustatten. Es ist aber durchaus auch möglich, dies nur für den peripheren Bereich 2 zu tun, so dass der zentrale Bereich 1 sich über die ganze Dicke des Präparates erstreckt, oder nur für den basalen Bereich 3, so dass sich der zentrale Bereich 1 über die ganze Fläche des Präparates erstreckt.

**Figur 2** zeigt eine weitere beispielhafte Ausführungsform des erfindungsgemässen Präparates. Es handelt sich um ein Präparat, mit dem der Innenkantenbereich eines Meniskus 7 ersetzbar ist. Das Präparat, das nur einen Knorpelteil A aufweist ist in der Figur in implantierter Position dargestellt. Da das Knorpelgewebe des Meniskus 7 nicht mit Knochengewebe verwachsen ist, wie dies für den Gelenkknorpel der Fall ist, muss das Präparat sich nur lateral im Knorpelgewebe des Meniskus integrieren und weist aus diesem Grunde nur einen zentralen Bereich 1 und einen peripheren Bereich 2 aber keinen basalen Bereich auf.

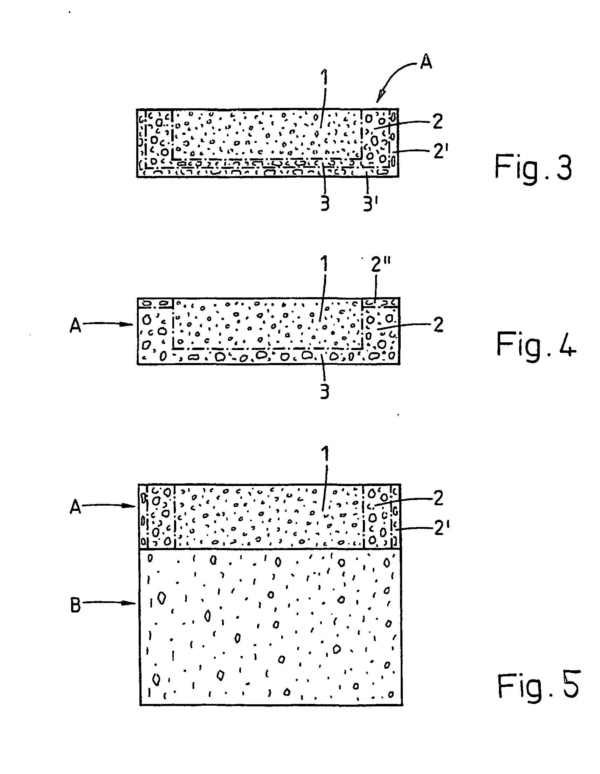

**Figuren 3 und 4** zeigen zwei weitere, beispielhafte Ausführungsformen des erfindungsgemässen Präparates im Schnitt. Die Präparate gemäss Figuren 2 und 3 entsprechen im wesentlichen dem in der Figur 1 dargestellten Präparat, das für die Reparatur eines enchondralen Defektes dient und nur einen Knorpelteil A hat, wobei aber der periphere Bereich 2 und der basale Bereich 3 (Figur 3) bzw. nur der periphere Bereich 2 (Figur 4) weitere Unterbereiche 2', 3, 2" aufweisen, die gegenüber dem Rest von peripherem oder basalem Bereich wiederum andere Eigenschaften haben. Vorteilhafte derartige Eigenschaften werden weiter unten beschrieben.

**Figur 5** zeigt ein Präparat, das zur Reparatur eines osteochondralen Defektes in einem Gelenk dient und das aus diesem Grunde nicht nur einen Knorpelteil A sondern auch einen am Knorpelteil A angeordneten Knochenteil B aufweist. Der Knorpelteil A ist ausgestaltet, wie dies beispielsweise in den Figuren 1, 3 und 4 dargestellt ist oder er weist nur einen peripheren Bereich 2 (gegebenenfalls mit Unterbereich 2') mit vom zentralen Bereich 1 verschiedenen Eigenschaften auf, während der zentrale Bereich 1 sich über die ganze Dicke des Knorpelteils A erstreckt, wie dies in der Figur 5 dargestellt ist.

Die Matrix des erfindungsgemässen Präparates bzw. des Knorpelteils A eines derartigen Präparates ist für eine Besiedlung mit Zellen geeignet, das heisst die Matrix weist eine offene Porosität auf und besteht aus einem entsprechend ausgewählten und/oder entsprechend beschichteten Material. Die Matrix ist mindestens teilweise biologisch abbaubar (resorbierbar). Sie besteht in an sich bekannter Weise beispielsweise vornehmlich aus Kollagen, aus Kollagen und Calziumphosphat oder aus einem resorbierbaren Polymer beispielsweise auf Milchsäure- und/oder Glykolsäurebasis. Die Matrix kann auch ein natürliches Produkt sein, das heisst sie kann beispielsweise aus denaturiertem Knorpelgewebe hergestellt sein.

Eine Matrix auf Kollagenbasis wird beispielsweise in an sich bekannter Weise hergestellt, indem Kollagenfasern nach der DHT-Methode (dehydro-thermalcrosslinking) vernetzt werden. Dabei wird eine gewünschte Porosität durch entsprechende Wahl der Verfahrensparameter erreicht. Insbesondere ergibt sich eine höhere Porosität, wenn in der Kollagen-Lösung, von der ausgegangen wird, die Kollagenkonzentration niedriger ist.

Wenn das erfindungsgemässe Präparat zusätzlich zum Knorpelteil A auch einen Knochenteil B aufweist, besteht dieser in an sich bekannter Art beispielsweise aus einem porösen Kalziumphosphat, das die Knochenregeneration positiv beeinflusst und während einer Heilungsphase resorbiert wird. Der Knochenteil kann auch aus einem resorbierbaren Kunststoff auf beispielsweise Milchsäure- und/oder Glykolsäurebasis bestehen.

Zur Herstellung eines erfindungsgemässen Präparates mit einem Knorpelteil mit einer Matrix aus Kollagen und einem Knochenteil mit einer Matrix aus porösem Calziumphosphat wird beispielsweise hergestellt dadurch, dass Kollagen auf den Calziumphosphatträger aufgebracht und teilweise in diesen eingesaugt wird und dass dann das Kollagen nach der DHT-Methode vernetzt wird.

Zellen, die im zentralen Bereich 1 angesiedelt sind oder sich ansiedeln sollen, haben in erster Linie die Aufgabe, die implantierte Matrix durch eine möglichst knorpelähnliche, interzelluläre Matrix zu ersetzen oder mit einer solchen Matrix zu ergänzen. Für diese Aufgabe eignen sich insbesondere Chondrozyten oder Zellen mit einem chondrogenen Potential, die in einem von Chondrozyten nicht weit entfernten Differenzierungsstadium sind. Es zeigt sich, dass im peripheren und basalen Bereich 2 und 3, wo es gilt, neu entstehendes Gewebe an vorhandenes Gewebe anzubinden, Zellen, die zwar ein chondrogenes Potential haben, die aber in einem weniger differenzierten Zustand sind als die Zellen des zentralen Bereichs, also beispielsweise pluripotente Stammzellen oder mesenchymale Stammzellen, bessere Resultate liefern als Chondrozyten. Solche, wenig differenzierte Zellen sind auch beweglicher und vermehrungsfähiger als die oben genannten Chondrozyten oder Chondrozyten-ähnlichen Zellen. Für ein erfolgreiches Verwachsen des Reparaturgewebes mit der subchondralen Knochenplatte eignen sich auch Zellen, die neben einem chondrogenen auch ein osteogenes Potential haben.

Die Besiedlung der verschiedenen Bereiche des Knorpelteils A des erfindungsgemässen Präparates mit Zellen in verschiedenen Differenzierungszuständen kann beispielsweise erzielt werden, indem von gleichen Zellen, beispielsweise von pluripotenten oder mesenchymalen Stammzellen ausgegangen wird, indem erst nur der zentrale Bereich besiedelt wird und die Zellen dann in vitro in der gewünschten Richtung differenziert werden, wozu den Zellen im Kulturmedium und/oder in der Matrix entsprechende, diese Differenzierung fördernde Faktoren zur Verfügung gestellt werden. Eine derartige Vorkultivierung beansprucht normalerweise einen Zeitraum von 2 bis 6 Wochen. Wenn die im zentralen Bereich angesiedelten Zellen den gewünschten Differenzierungsgrad erreicht haben, werden dieselben Zellen, die für die Vorkultivierung im zentralen Bereich angesiedelt wurden auch im peripheren und/oder basalen Bereich angesiedelt, wobei vorteilhafterweise in diesen Bereichen Faktoren vorgesehen werden, die die Zellvermehrung fördern, gegebenenfalls auch Faktoren, die die Differenzierung zu Chondrozyten fördern, die aber derart beispielsweise eingekapselt sind, dass sie erst verzögert freigesetzt werden.

Ein gleicher Effekt wie mit der oben beschriebenen Vorkultur der Zellen des zentralen Bereiches wird erzielt, wenn die verschiedenen Bereiche der Matrix mit verschiedenen Faktoren ausgestattet werden und alle Bereiche unmittelbar vor der Implantierung oder nach einer kurzen (z.B. zwei Tage) in vitro Kultur mit gleichen Zellen (z.B. Stammzellen) besiedelt werden oder die Matrix ohne Zellen implantiert wird und das Reparaturgewebe von aus umliegendem Gewebe eingewanderten Zellen gebildet wird. Der zentrale Bereich wird in diesem Falle mit Faktoren ausgestattet, die die gewünschte Differenzierung fördern, wobei die Faktoren in einer unmittelbar aktiven Form vorliegen. Der periphere und/oder basale Bereich wird mit Faktoren ausgestattet, die die Zellvermehrung fördern und in einer unmittelbar aktiven Form vorliegen. Gegebenenfalls können auch im peripheren und/oder basalen Bereich Faktoren vorgesehen werden, die eine gewünschte Differenzierung fördern und die verzögert freigesetzt werden.

Natürlicher Gelenkknorpel weist in äusseren Schichten mehr Zellen auf als in inneren, an die subchondrale Knochenplatte grenzenden Schichten. In Anlehnung an dieses Modell ist es vorteilhaft den basalen Bereich des erfindungsgemässen Präparates mit einer kleineren Zelldichte auszustatten als den zentralen Bereich oder darin gegebenenfalls gar keine Zellen anzusiedeln. Dieser basale Bereich wird dann von Zellen besiedelt, die aus dem äussersten Bereich der subchondralen Knochenplatte oder aus an die Defektstelle angrenzendem Grenzgebiet zwischen Knorpelgewebe und subchondraler Knochenplatte zuwandern und eine für dieses Grenzgebiet geeignete Differenzierung mitbringen. Um die Zellzuwanderung in den basalen Bereich zu fördern, kann dieser mit entsprechenden Faktoren ausgerüstet werden.

Für eine nur teilweise Besiedlung einer porösen Matrix mit Zellen wird beispielsweise eine entsprechende Zellsuspension auf die Matrixoberfläche aufgebracht und durch Saugen an der gegenüberliegenden Seite bis zur gewünschten Tiefe in die Matrix eingesaugt. Für die Besiedlung eines Oberflächenbereiches mit einer kleinen Tiefe genügt es gegebenenfalls, die Zellsuspension auf die entsprechende Matrixoberfläche aufzubringen.

Zur Herstellung einer mit Faktoren ausgestatteten Matrix können die Faktoren beispielsweise mit dem Matrixmaterial vermischt und die Mischung lyophilisiert werden. Es ist auch möglich, die Faktoren mit geeigneten Linkem an die Matrix zu binden oder sie in gelöster oder suspendierter Form der fertigen Matrix beizugeben.

Damit das Potential bezüglich Beweglichkeit und Vermehrungsfähigkeit der Zellen im peripheren und/oder basalen Bereich durch die Matrix nicht eingeschränkt wird, ist es vorteilhaft, diese in den genannten Bereichen mit einem höheren Anteil an Poren und/oder mit grösseren Poren auszugestalten als den zentralen Bereich.

Eine hoch-poröse Matrix insbesondere eine im peripheren Bereich hoch-poröse Matrix eignet sich wenig, um direkt mit dem umliegenden, nativen Knorpelgewebe vernäht zu werden, sie weist keine genügende Ausreisskraft auf. Aus diesem Grunde ist es vorteilhaft, gerade in diesem Falle mit anderen Mitteln für eine gute und einfach herstellbare, primäre Stabilität zu sorgen. Zu diesem Zwecke kann die Matrix in einem peripheren Bereich oder in einem äusseren Unterbereich (Figuren 2 und 4: 2') davon mit Fasern (z.B. Fasern aus Kollagen II) verstärkt werden oder einen höheren Anteil an Proteoglykanen aufweisen. Beide diese Massnahmen erlauben es, der Matrix in diesem Bereich eine für ein Annähen genügende mechanische Stabilität zu geben, ohne dass auf die hohe Porosität verzichtet werden muss, die gemäss den oben dargelegten Erkenntnissen gerade in diesem Bereich wichtig ist.

Es ist aber auch möglich, die Nähbarkeit durch Reduktion der Porosität zu erlangen, wenn dies in einem extrem kleinen Unterbereich getan wird, wie dies im Präparat gemäss Figur 3 mit dem Unterbereich 2" dargestellt ist. Eine dichtere Matrixstruktur im peripheren Bereich kann in einer durch Lyophilisation hergestellten Matrix, die beispielsweise aus Kollagen besteht, in der folgenden Weise erstellt werden: die Kollagenmasse wird auf eine Kühlfläche gegossen, wobei unmittelbar auf der Kühlfläche eine sehr schnell gefrierende und dadurch sehr fein strukturierte und dichte Schicht entsteht; diese Schicht wird nicht wie gemäss dem Stande der Technik vollständig entfernt sondern nur da, wo sie die äussere Oberfläche des zentralen Präparatbereiches bilden würde. Im peripheren Bereich dieser äusseren Oberfläche wird die dichtere Schicht belassen und bildet einen Ring eines dichteren Matrixmaterials, der einen für ein sicheres Annähen des Präparates am umliegenden Knorpelmaterial genügenden Ausreisswiderstand hat, auch wenn der Rest des peripheren Bereichs diesen Ausreisswiderstand nicht aufweist.

Um ein Präparat mit einer mechanischen Stabilität im peripheren Bereich, die nicht ausreicht, um das Präparat durch Nähen in der Defektstelle zu befestigen, ist es auch möglich, im peripheren und/oder basalen Bereich oder in äusseren Unterbereichen davon ein Verbindungsmittel in der Matrix zu integrieren. Dieses Verbindungsmittel ist in situ aktivierbar, beispielsweise mittels Laser. Ein derartiges Mittel ist beispielsweise ein Albumin-Kleber, dem Indocyanin-Grün (z.B. 0,1%) zugemischt ist und der mit einem Laser einer Wellenlänge von 808 nm aktiviert wird. Der Kleber kann in gelöster Form auf periphere und/oder basale Oberflächen des Präparats aufgebracht, durch Lyophilisation fixiert und nach der Implantation mit dem Laser aktiviert werden. Die mit dem Kleber ausgerüstete Präparatschicht hat vorteilhafterweise eine Dicke von nicht mehr als ca. 50 µm.

Zur Förderung der Integration des Präparates in das die Defektstelle umgebende Gewebe können dem peripheren und/oder basalen Präparatbereich auch Inhibitoren und/oder Antisense zugegeben werden, die einen auf die Implantation folgenden Abbau sowohl des entstehenden Reparaturgewebes als auch des ummittelbar daran angrenzenden ursprünglichen Gewebes verhindern oder abschwächen. Vorteilhafte derartige Zugaben sind beispielsweise Entzündungshemmer, Protease-Inhibitoren und/oder Apoptose-Inhibitoren.

Das Präparat ist also beispielsweise ein Präparat gemäss Figur 3, mit einer Matrix, die im zentralen Bereich eine geringere Porosität aufweist als im peripheren und basalen Bereich, dessen zentraler Bereich mit durch entsprechende Vorkultivierung weiter differenzierten Zellen besiedelt ist als der periphere und basale Bereich und dessen periphere und basale Oberflächen (Unterbereiche 2' und 3') einen mit Laser aktivierbaren Klebstoff aufweisen. Für ein ähnlich ausgerüstetes Präparat gemäss Figur 2 gilt in analoger Weise: Der zentrale Bereich weist eine geringere Porosität auf als der periphere Bereich und ist mit weiter differenzierten Zellen besiedet. Ferner ist ein äusserer Unterbereich des peripheren Bereichs 2 mit einem Klebstoff ausgerüstet.

Eine Ausführungsform des erfindungsgemässen Präparates, das erst unmittelbar vor der Implantation mit beispielsweise Stammzellen oder erst nach der Implantation mit aus der Umgebung zuwandernden Zellen besiedelt werden soll, weist die gleiche Matrix auf, wobei der zentrale Bereich sofort wirkende Faktoren, die eine gezielte Zelldifferenzierung fördern, enthält und der periphere und basale Bereich sofort wirkende Faktoren, die die Zellvermehrung fördern und gegebenenfalls verzögert wirkende Faktoren, die die Zelldifferenzierung fördern.

In einem Präparat mit einem Knorpelteil und einem Knochenteil kann nur der periphere Bereich in der oben beschriebenen Weise ausgerüstet sein und der basale Bereich ein Teil des zentralen Bereichs sein (Figur 5).

Je nach Menge und Art von zur Verfügung stehenden Zellen, je nach Art der Anwendung (enchondrale oder osteochondrale Defektstelle in Gelenken oder Meniskusdefekt. Human- oder Veterinär-Medizin, Reparaturstelle in belastetem oder weniger belastetem Gelenkbereich, Reparaturvermögen des nativen Gewebes um die Reparaturstelle etc.) reichen weniger der oben genannten Massnahmen für ein Präparat, das während der Heilungsphase gut im umliegenden Gewebe integriert wird und das mit einfachen Mitteln mit einer genügenden primären Stabilität implantiert werden kann.

## Patentansprüche

1. Präparat zur Reparatur von Knorpelgewebe, welches Präparat einen Knorpelteil (A) oder einen Knorpelteil (A) und einen Knochenteil (B) aufweist, wobei der Knorpelteil (A) eine offenporige, für eine Besiedlung mit Zellen geeignete Matrix aufweist, **dadurch gekennzeichnet, dass** ein peripherer Bereich (2) und/oder ein basaler Bereich (3) des Knorpel teils (A) Eigenschaften aufweist, durch die er sich von einem zentralen Bereich (1) unterscheidet, wobei in einem zentralen Bereich (1) des Knorpelteils A sofort wirkende, eine Zelldifferenzierung zu Chondrozyten fördernde Faktoren enthalten sind, die in einem peripheren und/oder basalen Bereich (2, 3) des Knorpelteils A fehlen oder in einer verzögert wirkenden Form enthalten sind, und dass der periphere Bereich (2) und/oder basale Bereich (3) Eigenschaften aufweist, die einer Integration des Präparates in der Defektstelle und/oder einer primären Stabilität des Präparates in der Defektstelle dienen und eine Einwanderung von Zellen nicht verhindern.

2. Präparat nach Anspruch 1, **dadurch gekennzeichnet, dass** das Präparat einen KnorpelteiL (A) und einen Knochenteil (B) aufweist.

3. Präparat nach Anspruch 2, **dadurch gekennzeichnet, dass** der Knochenteil (B) poröses Kalziumphosphat oder einen resorbierbaren Polymer enthält.

4. Präparat nach einem der Ansprüche 1-3, **dadurch gekennzeichnet, dass** die Matrix mindestens teilweise biologisch abbaubar ist.

5. Präparat nach einem der Ansprüche 1-4 , **dadurch gekennzeichnet, dass** die Matrix Kollagen, ein resorbierbares Polymer oder denaturiertes Knorpelgewebe enthält.

6. Präparat nach einem der Ansprüche 2-5, **dadurch gekennzeichnet, dass** die Matrix Kollagen enthält, welches vernetzt ist.

7. Präparat nach einem der Ansprüche 2-6, **dadurch gekennzeichnet, dass** die Matrix des Knorpelteils (A) mit Zellen besiedelt ist.

8. Präparat nach Anspruch 7, **dadurch gekennzeichnet, dass** die Matrix des Knorpelteils (A) in vitro mit Zellen besiedelt ist.

9. Präparat nach Anspruch 7 oder 8, **dadurch gekennzeichnet, dass** die Zellen im zentralen Bereich (1) weiter differenziert sind als die Zellen im peripheren und/oder basalen Bereich (2, 3).

10. Präparat nach einem der Ansprüche 7-9 , **dadurch gekennzeichnet, dass** die Zellen im zentralen Bereich (1) Chondrozyten sind oder Zellen mit einem Differenzierungsgrad, der demjenigen von Chondrocyten nahe ist, und dass die Zellen im peripheren und/oder basalen Bereich (2, 3) Stammzellen sind.

11. Präparat nach Anspruch 10, **dadurch gekennzeichnet, dass** die Stammzellen pluripotent sind.

12. Präparat nach Anspruch 10 oder 11, **dadurch gekennzeichnet, dass** die Zellen im peripheren und/oder basalen Bereich (2, 3) ein chondrogenes Potenzial oder ein chondrogenes und osteogenes Potential haben.

13. Präparat nach einem der Ansprüche 7-12, **dadurch gekennzeichnet, dass** der zentrale Bereich (1) der Matrix des Knorpelteils (A) mit einer grösscrcn Zelldichte besiedelt ist als der basale Bereich (3).

14. Präparat nach einem der Ansprüche 1 oder 2, **dadurch gekennzeichnet**, das die Matrix des Knorpelteils (A) in ihrem peripheren und/oder basalen Bereich Inhibitoren oder Antisense enthält.

15. Präparat nach einem der Ansprüche 1 oder 2, **dadurch gekennzeichnet. dass** die Matrix des Knorpelteiles (A) im peripheren und/oder basalen Bereich (2, 3) Entzündungshemmer, Protease-Inhibitoren und/oder Apoptose-Inhibitoren enthält.

16. Präparat nach einem der Ansprüche 1-15, **dadurch gekennzeichnet, dass** die Matrix des Knorpelteiles (A) in ihrem peripheren und/oder basalen Bereich ein Verbindungsmittel enthält.

17. Präparat nach Anspruch 16, **dadurch gekennzeichnet, dass** das Verbindungsmittel ein in situ aktivicrbarcs Verbindungsmittel ist.

18. Präparat nach einem der Ansprüche 1 und 4 bis 17, **dadurch gekennzeichnet, dass** es nur einen Knorpelteil (A) aufweist und dass es für die Reparatur einer enchondralen Defektstelle in Gelenkknorpel geeignet ist.

19. Präparat nach einem der Ansprüche 2 bis 17, **dadurch gekennzeichnet, dass** es einen Knorpelteil (A) und einen Knochenteil (B) aufweist und dass es für die Reparatur einer osteochondralen Defektstelle in Gelenkknorpel geeignet ist.

20. Präparat nach einem der Ansprüche 1 und 4 bis 17. **dadurch gekennzeichnet, dass** es nur einen Knorpelteil (A) aufweist und dass es für die Reparatur eines Meniskus (7) geeignet ist.

21. Präparat nach einem der Ansprüche 1 bis 20, **dadurch gekennzeichnet, dass** die Matrix des Knorpelteils (A) in mindestens einem Teil des peripheren Bereichs und/oder im basalen Bereich (2, 3) grössere und/oder mehr Poren aufweist als im zentralen Bereich (1).

## Claims

1. Preparation for repairing cartilage tissue, which preparation comprises a cartilage element (A) or a cartilage element (A) and a bone element (B), wherein the cartilage element (A) comprises an open-porous matrix suitable for being populated with cells, **characterized in that** a peripheral region (2) and/or a basal region (3) of the cartilage element (A) comprises characteristics rendering it different from a central region (1) of the cartilage element (A), wherein the central region (1) comprises immediately acting factors that promote cell differentiation to chondrocytes, said factors being absent from the peripheral and/or basal region (2, 3) or being comprised therein in a form with a delayed effect and wherein the peripheral and/or basal region (2, 3) comprises characteristics which serve integration of the preparation in the defective site and/or serve primary stability of the preparation in the defective site and do not prevent cell migration.

2. Preparation according to claim 1, **characterized in that** it comprises a cartilage element (A) and a bone element (B).

3. Preparation according to claim 2, **characterized in that** the bone element (B) comprises calcium phosphate or a resorbable polymer.

4. Preparation according to one of the claims 1 to 3, **characterized in that** the matrix is at least partially biodegradable.

5. Preparation according to one of the claims 1 to 4, **characterized in that** the matrix comprises collagen, a resorbable polymer or denatured cartilage tissue.

6. Preparation according to one of the claims 2 to 5, **characterized in that** the matrix comprises collagen which is cross linked.

7. Preparation according to one of the claims 2 to 6, **characterized in that** the matrix of the cartilage element (A) is populated with cells.

8. Preparation according to claim 7, **characterized in that** the matrix of the cartilage element (A) is populated with cells in vitro.

9. Preparation according to one of the claims 7 or 8, **characterized in that** the cells in the central region (1) are more differentiated than are the cells in the peripheral and/or basal region (2, 3).

10. Preparation according to one of the claims 7 to 9, **characterized in that** the cells in the central region (1) are chondrocytes or cells with a degree of differentiation close to that of chondrocytes, and that the cells in the peripheral and/or basal region (2, 3) are precursor cells.

11. Preparation according to claim 10, **characterized in that** the precursor cells are pluripotent.

12. Preparation according to one of the claims 10 or 11, **characterized in that** the cells in the peripheral and/or basal region (2, 3) have a chondrogenic or a chondrogenic and osteogenic potential.

13. Preparation according to one of the claims 7 to 12, **characterized in that** the matrix of the cartilage element (A) is populated with cells, wherein the cell density in the central region (1) is greater than the cell density in the basal region (3).

14. Preparation according to one of the claims 1 or 2, **characterized in that** the matrix of the cartilage element (A) contains inhibitors or antisenses in its peripheral and/or basal region (2, 3).

15. Preparation according to one of the claims 1 or 2, **characterized in that** the matrix of the cartilage element (A) contains inhibitors, protease inhibitors and/or apoptosis inhibitors in its peripheral and/or basal region (2, 3).

16. Preparation according to one of the claims 1 to 15, **characterized in that** the matrix of the cartilage element (A) contains a bonding agent in its peripheral and/or basal region (2, 3).

17. Preparation according to claim 16, **characterized in that** the activatable bonding agent is an in situ activatable bonding agent.

18. Preparation according to one of the claims 1 and 4 to 17, **characterized in that** it comprises only a cartilage element (A) and that it is suitable for the repair of an endochondral defect in a joint cartilage.

19. Preparation according to one of the claims 2 to 17, **characterized in that** it comprises a cartilage element (A) and a bone element (B) and that it is suitable for the repair of an osteochondral defect in a joint cartilage.

20. Preparation according to one of the claims 2 to 17, **characterized in that** it comprises only a cartilage element (A) and that it is suitable for the repair of a meniscus.

21. Preparation according to one of the claims 1 to 20, **characterized in that** at least in a part of its peripheral and/or basal regions (2, 3), the matrix of the cartilage element (A) comprises more and/or larger pores than in the central region (1).

## Revendications

1. Préparation destinée à la réparation de tissus cartilagineux, la préparation présentant une partie cartilagineuse (A) ou une partie cartilagineuse (A) et une partie osseuse (B), la partie cartilagineuse (A) présentant une matrice à pores ouverts apte à être colonisée par des cellules,
**caractérisée en ce que**
une portion périphérique (2) et/ou une portion de base (3) de la partie cartilagineuse (A) présentent des propriétés par lesquelles elles se distinguent d'une portion centrale (1), des facteurs agissant immédiatement et favorisant une différentiation cellulaire en chondrocytes étant repris dans une portion centrale (1) de la partie cartilagineuse (A) et étant absente dans une portion périphérique et/ou une portion de base (2, 3) de la partie cartilagineuse (A) ou y étant présente sous une forme à action ralentie et
**en ce que** la portion périphérique (2) et/ou la portion de base (3) présentent des propriétés qui servent à intégrer la préparation à l'emplacement défectueux et/ou à assurer la stabilité primaire de la préparation à l'emplacement défectueux et n'empêchent pas la pénétration de cellules.

2. Préparation selon la revendication 1, **caractérisée en ce que** la préparation présente une partie cartilagineuse (A) et une partie osseuse (B).

3. Préparation selon la revendication 2, **caractérisée en ce que** la partie osseuse (B) contient du phosphate de calcium poreux ou un polymère résorbable.

4. Préparation selon l'une des revendications 1 à 3, **caractérisée en ce que** la matrice est au moins en partie biodégradable.

5. Préparation selon l'une des revendications 1 à 4, **caractérisée en ce que** la matrice contient du collagène, un polymère résorbable ou du tissu cartilagineux dénaturé.

6. Préparation selon l'une des revendications 2 à 5, **caractérisée en ce que** la matrice contient du collagène réticulé.

7. Préparation selon l'une des revendications 2 à 6, **caractérisée en ce que** la matrice de la partie cartilagineuse (A) est colonisée par les cellules.

8. Préparation selon la revendication 7, **caractérisée en ce que** la matrice de la partie cartilagineuse (A) est colonisée in vitro par les cellules.

9. Préparation selon les revendications 7 ou 8, **caractérisée en ce que** les cellules présentes dans la portion centrale (1) sont davantage différenciées que les cellules présentes dans la portion périphérique et/ou la portion de base (2, 3).

10. Préparation selon l'une des revendications 7 à 9, **caractérisée en ce que** les cellules présentes dans la portion centrale (1) sont des chondrocytes ou des cellules dont le degré de différenciation est proche de celui des chondrocytes et **en ce que** les cellules de la portion périphérique et/ou de la portion basale (2, 3) sont des cellules souches.

11. Préparation selon la revendication 10, **caractérisée en ce que** les cellules souches sont pluripotentes.

12. Préparation selon l'une des revendications 10 ou 11, **caractérisée en ce que** les cellules de la portion périphérique et/ou de la portion de base (2, 3) ont un potentiel chondrogène ou un potentiel chondrogène et un potentiel ostéogène.

13. Préparation selon l'une des revendications 7 à 12, **caractérisée en ce que** la portion centrale (1) de la matrice de la partie cartilagineuse (A) est colonisée à une densité de cellules plus grande que la portion de base (3).

14. Préparation selon l'une des revendications 1 ou 2, **caractérisée en ce que** la matrice de la partie cartilagineuse (A) contient des inhibiteurs ou des anti-sens dans sa portion périphérique et/ou dans sa portion de base.

15. Préparation selon l'une des revendications 1 ou 2, **caractérisée en ce que** la matrice de la partie cartilagineuse (A) contient dans sa portion périphérique et/ou dans sa portion basale (2, 3) des inhibiteurs d'inflammation, des inhibiteurs de protéase et/ou des inhibiteurs d'apoptose.

16. Préparation selon l'une des revendications 1 à 15, **caractérisée en ce que** la matrice de la partie cartilagineuse (A) contient un agent de liaison dans sa portion périphérique et/ou dans sa portion basale.

17. Préparation selon la revendication 16, **caractérisée en ce que** l'agent de liaison est un agent de liaison apte à être activé in situ.

18. Préparation selon l'une des revendications 1 et 4 à 17, **caractérisée en ce qu'**elle ne présente qu'une seule partie cartilagineuse (A) et **en ce qu'**elle convient pour réparer un emplacement endochondral défectueux du cartilage d'une articulation.

19. Préparation selon l'une des revendications 2 à 17, **caractérisée en ce qu'**elle présente une partie cartilagineuse (A) et une partie osseuse (B) et **en ce qu'**elle convient pour réparer un emplacement ostéochondral défectueux dans le cartilage d'une articulation.

20. Préparation selon l'une des revendications 1 et 4 à 17, **caractérisée en ce qu'**elle présente uniquement une partie cartilagineuse (A) et **en ce qu'**elle convient pour la réparation d'un ménisque (7).

21. Préparation selon l'une des revendications 1 à 20, **caractérisée en ce que** la matrice de la partie cartilagineuse (A) présente dans au moins une partie de sa portion périphérique et/ou de sa portion basale (2, 3) des pores plus grands et/ou plus nombreux que dans sa portion centrale (1).
